(19) Europäisches Patentamt
European Patent Office
Office européen des brevets

(11) Veröffentlichungsnummer: **0 652 193 A1**

# (12) EUROPÄISCHE PATENTANMELDUNG

(21) Anmeldenummer: **94116868.4**

(51) Int. Cl.[6]: **C07C 2/34**

(22) Anmeldetag: **26.10.94**

(30) Priorität: **05.11.93 DE 4337782**

(43) Veröffentlichungstag der Anmeldung:
**10.05.95 Patentblatt 95/19**

(84) Benannte Vertragsstaaten:
**BE DE ES FR GB IT NL**

(71) Anmelder: **BASF Aktiengesellschaft**
**Carl-Bosch-Strasse 38**
**D-67063 Ludwigshafen (DE)**

(72) Erfinder: **Marczinke, Bernd Lothar, Dr.**
**Wormser Landstr. 17**
**D-67346 Speyer (DE)**
Erfinder: **Langhauser, Franz, Dr.**
**Salinenstr. 103**
**D-67098 Bad Dürkheim (DE)**
Erfinder: **Müller, Hans-Joachim, Dr.**
**Pfortmüllerstr. 52**
**D-67269 Grünstadt (DE)**
Erfinder: **Seelert, Stefan, Dr.**
**Mathäus-Merian-Ring 24a**
**D-67227 Frankenthal (DE)**

## (54) Verfahren zur Herstellung von Oligomeren von C2- bis C12-Alk-1-enen.

(57) Verfahren zur Herstellung von Oligomeren von $C_2$- bis $C_{12}$-Alk-1-enen, dadurch gekennzeichnet, daß
a) $C_2$- bis $C_{12}$-Alk-1-ene in Gegenwart eines Katalysatorsystems, das als aktive Bestandteile
A) einen feinteiligen Träger,
B) eine Komplexverbindung von Metallen der IV. oder V. Nebengruppe des Periodensystems
und
C) einen Cokatalysator
enthält, oligomerisiert werden
und
b) anschließend das Katalysatorsystem mechanisch abgetrennt wird.

EP 0 652 193 A1

Die vorliegende Erfindung betrifft ein Verfahren zur Herstellung von Oligomeren von $C_2$- bis $C_{12}$-Alk-1-enen.

Unter ausgewählten Verfahrensbedingungen ist es unter Verwendung von Übergangsmetallkomplexen möglich, Alk-1-ene zu niedermolekularen Verbindungen umzusetzen. So beschreibt EP-A 268 214 ein Verfahren zur Herstellung von Propenoligomeren mit Hilfe von, aus peralkylierten Cyclopentadienylliganden hergestellten, Titanocen, Zirkonocen und Hafnocen-Katalysatoren und Aluminoxan-Cokatalysatoren.

Die EP-A 257 696 offenbart ein Verfahren zur Dimerisierung von $\alpha$-Olefinen, wie Propen, mit Hilfe von Zirkonocen- und Hafnocen-Katalysatoren.

In der DE-A 42 05 932 wird ein Verfahren zur Übergangsmetallkomplex-katalysierten Oligomerisierung von Propen in flüssiger Phase zu Propenoligomeren mit einem hohen Gehalt an endständigen Doppelbindungen beschrieben. Dabei wird in flüssigem Propen mit Lösungen von Cyclopentadienylkomplexen des Zirkons oder Hafniums in Gegenwart von Methylalumoxan als Cokatalysator in homogener Phase gearbeitet.

Nachteil dieser Verfahren ist, daß nach Ende der Reaktion die im anfallenden Oligomerengemisch gelösten Katalysatorbestandteile in aufwendiger Weise abgetrennt werden müssen.

Dies geschieht z.B. durch Abdestillieren der flüchtigen Oligomeren nach Abschluß der Reaktion aus dem Oligomerengemisch oder durch Versetzen dieses Gemischs mit einer wäßrigen Lösung eines Komplexbildners wie z.B. EDTA (Ethylendiamintetraessigsäure). Der Nachteil bei der Abdestillation liegt darin, daß während der Destillation unerwünschte Nebenreaktionen, vor allem hervorgerufen durch den Cokatalysaotr, auftreten. Bei der Zugabe der wäßrigen EDTA-Lösung ist es hingegen notwendig, die wäßrige Phase wieder abzutrennen, die organische Phase zu filtrieren und anschließen zu trocknen, d.h. dies ist verfahrenstechnisch sehr aufwendig.

Aufgabe der vorliegenden Erfindung war es daher, ein heterogenkatalysiertes Verfahren zur Herstellung von Oligomeren von $C_2$- bis $C_{12}$-Alk-1-enen zur Verfügung zu stellen, welches verfahrenstechnische Vorteile aufweist und insbesondere die aufwendige Aufarbeitung der Oligomeren von $C_2$- bis $C_{12}$-Alk-1-enen durch thermische Trennverfahren wie Destillation überflüssig macht.

Demgemäß wurde ein Verfahren zur Herstellung von Oligomeren von $C_2$- bis $C_{12}$-Alk-1-enen gefunden, welches dadurch gekennzeichnet ist, daß

a) $C_2$- bis $C_{12}$-Alk-1-ene in Gegenwart eines Katalysatorsystems, das als aktive Bestandteile

A) einen feinteiligen Träger,

B) eine Komplexverbindung von Metallen der IV. oder V. Nebengruppe des Periodensystems und

C) einen Cokatalysator

enthält, oligomerisiert werden und

b) anschließend das Katalysatorsystem mechanisch abgetrennt wird.

Das erfindungsgemäße Verfahren wird zur Herstellung von Oligomeren von $C_2$- bis $C_{12}$-Alk-1-enen, bevorzugt von $C_2$- bis $C_8$-Alk-1-enen verwendet. Unter dem Begriff Oligomere sind sowohl die Homooligomeren als auch die Cooligomeren von $C_2$- bis $C_{12}$-Alk-1-enen zu verstehen. Bevorzugte Homooligomere sind Ethylen-, Propylen-, But-1-en- oder 4-Methylpent-1-en-oligomere, bevorzugte Cooligomere sind Ethylen-cooligomere, wobei als Comonomere insbesondere $C_3$- bis $C_6$-Alk-1-ene eingesetzt werden, vorzugsweise Propylen. Die Menge an Ethylen beträgt hierbei 0,01 bis 7 Mol-%, bevorzugt 0,01 bis 5 Mol-%, bezogen auf die anderen Alk-1-ene.

Bei dem erfindungsgemäßen Verfahren werden in der Stufe a) $C_2$- bis $C_{12}$-Alk-1-ene in Gegenwart eines Katalysatorsystems oligomerisiert. Als Komponente A) enthält dieses Katalysatorsystem einen feinteiligen Träger, der bevorzugt einen Teilchendurchmesser im Bereich von 1 bis 200 $\mu$m aufweist, insbesondere von 30 bis 70 $\mu$m. Geeignete Trägermaterialien sind beispielsweise Kieselgele, bevorzugt solche der Formel $SiO_2 \cdot aAl_2O_3$, worin a für eine Zahl im Bereich von 0 bis 2 steht, vorzugsweise 0 bis 0,5; dies sind also Alumosilikate oder Siliciumdioxid. Derartige Produkte sind im Handel erhältlich, z.B. Silica Gel 332 der Fa. Grace. Bevorzugt sind auch Kieselgele, die einen Wassergehalt von 0,5 bis 8 Gew.-% aufweisen.

Als Komponente B) werden Komplexverbindungen von Metallen der IV. oder V. Nebengruppe das Periodensystems eingesetzt, vorzugsweise solche, bei denen das Metallatom über $\Pi$-Bindungen mit ungesättigten cyclischen Kohlenwasserstoffresten verbunden ist, beispielsweise substituierte oder unsubstituierte Cyclopentadienylgruppen. Weiterhin sind die bevorzugt eingesetzten Komplexverbindungen dadurch gekennzeichnet, daß das Metallatom noch mit weiteren Liganden, insbesondere mit Fluor, Chlor, Brom, Jod, Wasserstoff oder Methyl verknüpft sein kann.

Besonders geeignete Komplexverbindungen lassen sich durch folgende allgemeine Formel I kennzeichnen:

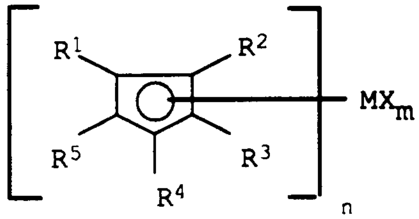

I

in der

R[1] bis R[5] Wasserstoff oder $C_1$- bis $C_4$-Alkyl,
M Titan, Zirkonium oder Hafnium,
X Fluor, Chlor, Brom, Jod, Wasserstoff oder Methyl bedeuten

und

n, m für die Zahlen 1, 2 oder 3 stehen, wobei die Summe n + m 4 ist.

Bevorzugt sind hier Bis(cyclopentadienyl)zirkoniumdichlorid, Bis(pentamethylcyclopentadienyl)-zirkoniumdichlorid, Bis(n-butylcyclopentadienyl)zirkoniumdichlorid, Bis(methylcyclopentadienyl)-zirkoniumdichlorid und Bis(cyclopentadienyl)zirkoniumdimethyl.

Weiterbin sind

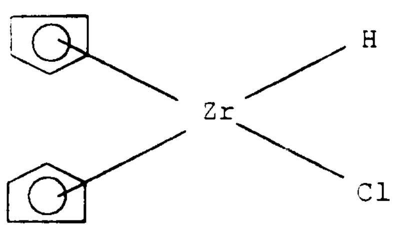

und

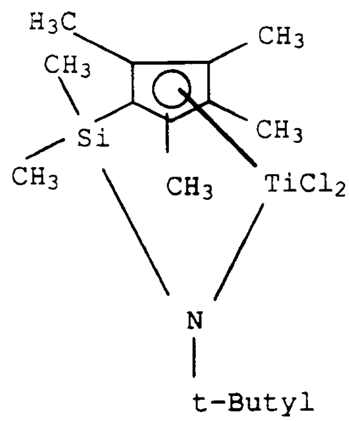

geeignet.

Die Synthese derartiger Komplexverbindungen kann nach an sich bekannten Methoden erfolgen, wobei die Umsetzung der entsprechend substituierten, cyclischen Kohlenwasserstoffanionen mi Halogeniden von Titan, Zirkonium, Hafnium, Vanadium, Niob oder Tantal bevorzugt ist. Beispiele für entsprechende Herstellungsverfahren sind u.a. im Journal of Organometallic Chemistry, 369 (1989), 359-370 beschrieben.

Als Cokatalysatoren C) eignen sich Aluminiumalkyle wie Aluminiumtrimethyl oder Aluminiumtriethyl oder offenkettige oder cyclische Alumoxanverbindungen der allgemeinen Formeln II oder III

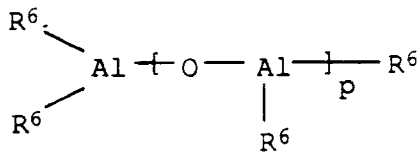

II

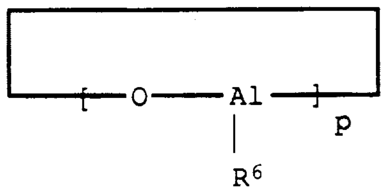

III

wobei

$R^6$ eine $C_1$- bis $C_4$-Alkylgruppe, bevorzugt Methyl oder Ethyl bedeutet und

p für eine ganze Zahl von 5 bis 30, bevorzugt 10 bis 25 steht.

Die Herstellung dieser oligomeren Alumoxanverbindungen erfolgt üblicherweise durch Umsetzung einer Lösung von Trialkylaluminium mit Wasser und ist u.a. in der EP-A 284 708 und der US-A 4,794,096 beschrieben.

In der Regel liegen die dabei erhaltenen oligomeren Alumoxanverbindungen als Gemische unterschiedlich langer, sowohl linearer als auch cyclischer Kettenmoleküle vor, so daß p als Mittelwert anzusehen ist. Die Alumoxanverbindungen können auch im Gemisch mit anderen Metallalkylen, bevorzugt mit Aluminiumalkylen vorliegen.

In Verbindung mit Metallocendialkylen, beispielsweise Bis(cyclopentadienyl)zirkoniumdimethyl, können auch Lewis-Säuren, beispielsweise Tris(pentafluorophenyl)bor eingesetzt werden.

Zweckmäßigerweise wird bei der Oligomerisierung nur ein Katalysatorsystem eingesetzt, es ist aber auch möglich, Mischungen verschiedener Katalysatorsysteme zu verwenden.

Zur Herstellung der Katalysatorsysteme geht man vorzugsweise so vor, daß auf den Träger (Komponente A)) $10^{-2}$ bis $10^{-6}$ mol/g Träger, bevorzugt $10^{-3}$ bis $10^{-5}$ mol/g Träger der Metallocen-Komponente (Komponente B)) aufgebracht werden. Die Cokatalysatoren (Komponente C)), die üblicherweise zusammen mit der Komponente B) eingesetzt werden, werden im molaren Verhältnis Al:M von $10^2$:1 bis $10^4$:1, vorzugsweise 200:1 bis 1000:1, insbesondere 300:1 bis 600:1 eingesetzt.

Die Oligomerisierung erfolgt im allgemeinen bei Temperaturen von 40 bis 150 °C, bevorzugt 45 bis 90 °C und Drücken von 15 bis 120 bar, bevorzugt 20 bis 80 bar. Es kann sowohl chargenweise, z.B. in Rührautoklaven, als auch kontinuierlich, z.B. in Rohrreaktoren gearbeitet werden.

Die Oligomerisierung läßt sich in der Gasphase, in einer Suspension, in flüssigen Monomeren oder in inerten Lösungsmitteln durchführen. Vorzugsweise wird in flüssiger Phase und in einem Lösungsmittel, zweckmäßigerweise unter Verwendung geringer Mengen an Lösungsmittel, vorzugsweise einem aliphatischen oder aromatischen Kohlenwasserstoff wie Benzol, Toluol, Xylole, n-Hexan, n-Heptan, Isobutan, Petrolether, Ligroin oder deren Mischungen oligomerisiert. Vorzugsweise werden Hexan, Toluol oder Xylole verwendet. Die Mengen an Lösungsmittel betragen 0 bis 80 Gew.-%, vorzugsweise 0 bis 50 Gew.-%, bezogen auf das erhaltene Oligomerisat.

Üblicherweise erfolgt die Oligomerisierung über einen Zeitraum von 5 bis 600 Minuten, vorzugsweise 10 bis 500 Minuten.

Nachdem im erfindungsgemäßen Verfahren in der Stufe a) $C_2$- bis $C_{12}$-Alk-1-ene in Gegenwart dieser Katalysatorsysteme oligomerisiert wurden, wird anschließend in der Stufe b) das Katalysatorsystem mechanisch abgetrennt. Mechanische Trennverfahren wie Sieben, Klassieren, Sortieren, Entschlämmen, Entstauben, Filtrieren, Zentrifugieren oder Pressen sind bekannt und beispielsweise in W. Brötz, A. Schönbucher, Technische Chemie I, S. 97-125 beschrieben. Als besonders geeignet hat sich das Filtrieren und Zentrifugieren erwiesen. Als Filter können Trichter mit Papierfiltern verwendet werden, sofern die zu filtrierende Menge nicht allzu groß ist. Gängige Filter wie Nutschen oder Trommelfilter können ebenfalls eingesetzt werden, ebenso wie die üblichen Zentrifugen.

Durch das verfahrenstechnisch einfache mechanische Abtrennen der Katalysatorsysteme erhält man Oligomere von $C_2$- bis $C_{12}$-Alk-1-ene, die keine signifikanten Mengen an Katalysatorsystemen mehr

aufweisen. Anschließend kann man beispielsweise über fraktionierte Destillation die Zusammensetzung der Oligomeren bestimmen.

Die nach dem erfindungsgemäßen Verfahren hergestellten Oligomere von $C_2$- bis $C_{12}$-Alk-1-enen können durch Funktionalisierung der endständigen Doppelbindungen z.B. zu Alkoholen, Aldehyden, Säuren oder Estern umgesetzt werden, die in vielfältiger Weise technisch Verwendung finden.

Beispiele

Herstellung eines Trägermaterials

Zu einer Suspension von 20,2 g Kieselgel (Fa. Grace, SG 332, Teilchendurchmesser 20-45 µm) in 200 ml Heptan wurden bei Raumtemperatur während 30 min 56 ml einer Lösung von 6,4 g Triethylaluminium in 48 ml Heptan zugetropft. Dabei stieg die Temperatur auf 44 °C an. Nach 18 Stunden Rühren bei Raumtemperatur wurde abfiltriert, zweimal mit je 30 ml Heptan und zweimal mit je 30 ml Pentan gewaschen und anschließend am Ölpumpenvakuum getrocknet.
Ausbeute: 22 g

Trägerung eines Katalysators

Zu 284,2 mg (≙ 0,97 mmol) Bis(cyclopentadienyl)zirkoniumdichlorid (Fa. Aldrich) wurden unter Argonatmosphäre 261,4 ml (≙ 0,4 mol) einer Lösung von Methylalumoxan in Toluol (1,53 molar, Fa. Witco) gegeben und 15 Minuten gerührt. Anschließend wurden 10,1 g des Trägermaterials zugegeben (≙ 0,1 mmol Metallocen/g Träger) und weitere 30 Minuten gerührt. Zuletzt wurde das Lösungsmittel bei Raumtemperatur während 4 Stunden am Ölpumpenvakuum entfernt. Es entstand ein gelber Feststoff.
Ausbeute: 33 g (90 % der Theorie)

Beispiel 1: Herstellung eines Propylen-Homooligomeren

2540 mg des geträgerten Katalysators wurden mit 30 ml Heptan (ohne weiteren Zusatz von Aluminiumtrialkylen) in einen 2 l-Stahlautoklaven gegeben und 14 mol Propylen 90 Minuten lang oligomerisiert. Nach Beendigung der Reaktion wurde das flüssige Propylen-Homooligomere ausgetragen und mittels eines Trichters, in den ein handelsüblicher Papierfaltenfilter (300 mm Durchmesser) eingelegt war, abfiltriert. Das erhaltene Filtrat enthielt keine signifikanten Mengen des geträgerten Katalysators. Anschließend wurde das Propylen-Homooligomere im Ölpumpenvakuum bei 2 mbar fraktioniert destilliert, um die im Temperaturbereich von 30 bis 80 °C flüchtigen Komponenten abzutrennen.

Beispiel 2: Herstellung eines Propylen-Ethylen-Cooligomeren

Es wurde analog zu Beispiel 1 gearbeitet, jedoch wurden 2600 mg des geträgerten Katalysators eingesetzt und 13,5 mol Propylen und 0,5 mol Ethylen.

Vergleichsbeispiel V1: Herstellung eines Propylen-Homooligomeren

Anstelle der heterogenen Katalyse wie in den Beispielen 1 und 2 wurde eine homogene Katalyse durchgeführt. Hierzu wurden in einem 2 l-Stahlautoklaven 30 ml (≙ 48 mmol) einer Lösung von Methylalumoxan in Toluol (1,6 molar) vorgelegt, 14 mol Propylen eingeleitet und auf Reaktionstemperatur erwärmt. Anschließend wurden 21,1 mg (≙ 0,072 mmol) Bis(cyclopentadienyl)zirkoniumdichlorid hinzugegeben und 90 Minuten oligomerisiert. Nach Beendigung der Reaktion wurde das flüssige Propylen-Homooligomere ausgetragen und zur Desaktivierung des Katalysators mit 100 ml 3 gew.-%iger wäßriger Ethylendiamintetraessigsäure-Lösung versetzt. Dann wurde die organische Phase mittels Phasentrennung abgetrennt. Anschließend wurde der Niederschlag nach dem in Beispiel 1 beschriebenen Verfahren abfiltriert. Das Produkt wurde über ein Molekularsieb getrocknet und fraktioniert destilliert.

Vergleichsbeispiel V2: Herstellung eines Propylen-Homooligomeren

Es wurde eine weitere homogen katalysierte Oligomerisierung im Autoklaven entsprechend Vergleichsbeispiel V1 mit einem Katalysator aus 30 ml (≙ 48 mmol) einer Lösung von Methylalumoxan in Toluol (1,6 molar), 21,5 mg (≙ 0,073 mmol) Bis(cyclopentadienyl)zirkoniumdichlorid und 14 mol Propylen durchgeführt.

Dabei wurde jedoch das aus dem Autoklaven ausgetragene Produkt direkt, ohne weitere Aufarbeitung, fraktioniert destilliert.

Die Reaktionsbedingungen und die Eigenschaften der Oligomeren sind in der Tabelle zusammengestellt.

Die Produktivität [g/g Kat•h] bezieht sich auf die Menge an eingesetztem Bis(cyclopentadienyl)-zirkoniumdichlorid. Die Menge an Destillat entspricht der Menge an Flüssigkeit, die im Temperaturbereich von 30 bis 80 °C gewonnen wurde.

Bei Vergleichsbeispiel V1 war die Filtrationszeit deutlich länger als in den Beispielen 1 und 2, bei Vergleichsbeispiel V2 war die Menge an Destillat geringer.

Tabelle

| Beispiel | Temperatur [°C] | Druck [bar] | Ausbeute an Oligomeren [g] | Produktivität [g/g Kat·h] | Filtrations-zeit [min/100 ml] | Menge an De-stillat [g] |
|---|---|---|---|---|---|---|
| 1 | 60 | 22 | 400 | 12300 | 3 | 81 |
| 2 | 50 | 20 | 670 | 20400 | 2 | 75 |
| V1 | 60 | 22 | 550 | 17400 | 20 | 110 |
| V2 | 60 | 22 | 570 | 17700 | - | 30 |

## Patentansprüche

**1.** Verfahren zur Herstellung von Oligomeren von $C_2$- bis $C_{12}$-Alk-1-enen, dadurch gekennzeichnet, daß
a) $C_2$- bis $C_{12}$-Alk-1-ene in Gegenwart eines Katalysatorsystems, das als aktive Bestandteile

A) einen feinteiligen Träger,
B) eine Komplexverbindung von Metallen der IV. oder V. Nebengruppe des Periodensystems und
C) einen Cokatalysator enthält, oligomerisiert werden

und
b) anschließend das Katalysatorsystem mechanisch abgetrennt wird.

2. Verfahren nach Anspruch 1, dadurch gekennzeichnet, daß als Komponente A) Träger mit einem Teilchendurchmesser im Bereich von 1 bis 200 µm eingesetzt werden.

3. Verfahren nach den Ansprüchen 1 bis 2, dadurch gekennzeichnet, daß als Komponente A) Kieselgele eingesetzt werden.

4. Verfahren nach den Ansprüchen 1 bis 3, dadurch gekennzeichnet, daß als Komponente B) Metallocenkomplexe der allgemeinen Formel I

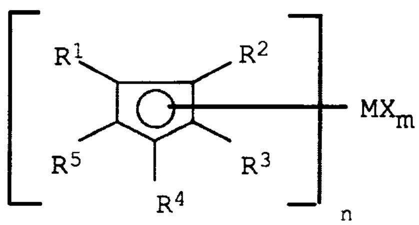

I

in der
$R^1$ bis $R^5$ Wasserstoff oder $C_1$- bis $C_4$-Alkyl,
M Titan, Zirkonium oder Hafnium,
X Fluor, Chlor, Brom, Jod, Wasserstoff oder Methyl bedeuten
und
n,m für die Zahlen 1, 2 oder 3 stehen, wobei die Summe n + m 4 ist
eingesetzt werden.

5. Verfahren nach den Ansprüchen 1 bis 4, dadurch gekennzeichnet, daß als Komponente C) Aluminiumalkyle oder offenkettige oder cyclische Alumoxanverbindungen der allgemeinen Formel II oder III

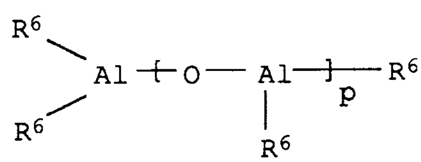

II

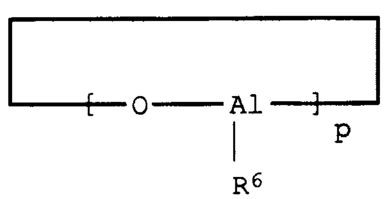

III

wobei
$R^6$ eine $C_1$- bis $C_4$-Alkylgruppe bedeutet und p für eine ganze Zahl von 5 bis 30 steht,
eingesetzt werden.

6. Verfahren nach den Ansprüchen 1 bis 5, dadurch gekennzeichnet, daß die Oligomerisierung a) bei Temperaturen von 40 bis 150 ° C und Drücken von 15 bis 120 bar erfolgt.

7. Verfahren nach den Ansprüchen 1 bis 6, dadurch gekennzeichnet, daß die Oligomerisierung a) in flüssiger Phase erfolgt.

8. Verfahren nach den Ansprüchen 1 bis 7, dadurch gekennzeichnet, daß die mechanische Abtrennung b) durch Abfiltrieren oder Zentrifugieren erfolgt.

Europäisches Patentamt

# EUROPÄISCHER RECHERCHENBERICHT

Nummer der Anmeldung
EP 94 11 6868

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | US-A-5 087 788 (FENG-JUNG WU ET AL)<br>--- | | C07C2/34 |
| A | EP-A-0 366 212 (SHELL INTERNATIONALE RESEARCH MAATSCHAPPIJ)<br>----- | | |

| RECHERCHIERTE SACHGEBIETE (Int.Cl.6) |
|---|
| C07C |

Der vorliegende Recherchenbericht wurde für alle Patentansprüche erstellt

| Recherchenort | Abschlußdatum der Recherche | Prüfer |
|---|---|---|
| DEN HAAG | 25. Januar 1995 | Van Geyt, J |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P04C03)